(19) Europäisches Patentamt
European Patent Office
Office européen des brevets

(11) Veröffentlichungsnummer: **0 069 894**
**B1**

(12) # EUROPÄISCHE PATENTSCHRIFT

(45) Veröffentlichungstag der Patentschrift: **10.04.85**

(51) Int. Cl.⁴: **C 07 K 5/06**, A 61 K 37/02

(21) Anmeldenummer: **82105590.2**

(22) Anmeldetag: **25.06.82**

(54) **Optisch aktives Dipeptid, seine pharmazeutisch verträglichen Salze, Verfahren zur Herstellung und diese enthaltende pharmazeutische Präparate.**

(30) Priorität: **15.07.81 DE 3127930**

(43) Veröffentlichungstag der Anmeldung: **19.01.83 Patentblatt 83/3**

(45) Bekanntmachung des Hinweises auf die Patenterteilung: **10.04.85 Patentblatt 85/15**

(84) Benannte Vertragsstaaten: **AT BE CH FR GB IT LI LU NL SE**

(56) Entgegenhaltungen:
**EP - A - 0 013 891**

**Chemical Abstracts Band 39, Nr. 10 20. Mai 1945 Columbus, Ohio, USA W.C. CRAIG et al. "Interaction of alpha-4-morpholinylpropiophenone with KCN and (NH4)2CO3." Spalte 2072, Abschnitt 4 bis 8**

(73) Patentinhaber: **A. Nattermann & Cie. GmbH, Nattermannallee 1, D-5000 Köln 30 (DE)**

(72) Erfinder: **Biedermann, Jürgen, Dr., Kirschenweg 14, D-5024 Pulheim-Stommeln (DE)**
Erfinder: **Etschenberg, Eugen, Dr., Hirseweg 10, D-5000 Köln 41 (DE)**
Erfinder: **Friehe, Hugo, Dr., Am Burgfeld 94, D-5042 Erftstadt-Lechenich (DE)**
Erfinder: **Scheef, Wolfgang, Dr. Dr., Andreasstrasse 12-14, D-5300 Bonn 2 (DE)**
Erfinder: **Winkelmann, Johannes, Dr., Frankfurter Strasse 269, D-5000 Köln 90 (DE)**

(74) Vertreter: **Redies, Bernd, Dr. rer. nat. et al, Redies, Redies, Türk & Gille, Patentanwälte Brucknerstrasse 20, D-4000 Düsseldorf 13 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

**Beschreibung**

Die vorliegende Erfindung betrifft das neue optisch aktive Dipeptid D-2-Phenylgylcyl-D-2-phenylglycin und seine pharmakologisch verträglichen Salze und Säureadditionssalze sowie deren Verwendung als tumorgewebs- und gewebsauflösendes Arzneimittel.

Die tumorgewebs- und gewebsauflösende Wirkung verschiedener Substanzen ist an sich bekannt. Die zur Behandlung der verschiedenen Krebsarten betriebene Chemotherapie mit strukturell recht unterschiedlichen Cytostatika, wie z.B. Adriamycin, Bleomycin, Vinblastin, Vincristin, Cisplatin und den Oxazaphosphorinen Cyclophosphamid und Ifosfamid zeigt neben Teilerfolgen immer noch stark ausgeprägte Nebenwirkungen, wie z.B. Anorexie, Diarrhoe, Leukopenie, Thrombozytopenie, Nausea, Erbrechen, Haarverlust. Obwohl in letzter Zeit erfolgreiche Versuche zur Reduzierung der Nebenwirkungen unternommen wurden, wie z.B. die Kombination der Oxazaphosphorine mit Mesna, einer Mercaptoethylsulfonsäure, hat die Auffindung neuer, vom Organismus besser tolerierter Substanzen mit direkter oder indirekter Wirkung gegen Krebs nach wie vor höchsten Stellenwert.

In der DE-OS 2 901 667 werden Dipeptide von essentiellen Aminosäuren vorgeschlagen. Die Toxizität dieser Verbindungen ist mit 300 mg/kg i.v. (Nager) aber immer noch recht hoch. In der gleichen Offenlegung wird der Einsatz der wenig toxischen Dehydrooligopeptide (DE-OS 2 659 154) erwähnt, die aber einige Nachteile aufweisen. Diese Nachteile der Dehydrooligopeptide wurden im reproduzierten Tierversuch als Auslösung starker Schmerzzustände bei den Versuchstieren erkannt.

Überraschenderweise wurde nun gefunden, dass man in dem neuen optisch aktiven Dipeptid D-2-Phenylglycyl-D-2-phenylglycin eine Substanz hat, deren Toxizität um den Faktor 3 kleiner ist bei hervorragenden nekrogenen und makrophagen-aktivierenden Eigenschaften. Dies ist um so mehr überraschend, als von den 9 Kombinationsmöglichkeiten des D-, L- und DL-2-Phenylglycins zu Dipeptiden das Racemat DL-2-Phenylglycyl-DL-2-phenylglycin, welches neben 2,5-Dimethyl-3,6-diphenyl-pyrazin beim Erhitzen von (±)-2-Morpholino-1-phenylpropanon-1-hydrochlorid mit Kaliumcyanid und Ammoniumcarbonat in wässrigem Ethanol auf 105°C erhalten wird [Craig W.C. und Henze H.R., J. Org. Chem. 10 (1945), 10-15], nur sehr geringe Wirkung zeigt und das L-2-Phenylglycyl-L-2-phenylglycin wirkungslos ist.

Nach den Untersuchungsbefunden ist die erfindungsgemässe Verbindung D-2-Phenylglycyl-D-2-phenylglycin-hydrochlorid für das Indikationsgebiet Tumor-Therapie am besten geeignet, vorzugsweise bei intratumoraler Application. Die vorliegende Erfindung betrifft D-2-Phenylglycyl-D-2-phenylglycin und seine pharmazeutisch verträglichen Salze bzw. Säureadditionssalze, wie z.B. Dihydrogenphosphat, Hydrogensulfat. Besonders hervorzuheben sind das D-2-Phenylglycyl-D-2-phenylglycin-hydrochlorid und das D-2-Phenylglycyl-D-2-phenylglycin-hydrobromid, ganz besonders das Hydrochlorid.

Die Darstellung der erfindungsgemässen Verbindung erfolgt in an sich bekannter Weise nach den üblichen, in der Peptidchemie gebräuchlichen Methoden, wie z.B. der Azid-Methode, nach dem Verfahren der gemischten Anhydride, nach der Carbodiimid-Methode, nach dem variantenreichen Verfahren der Aktivester oder nach der Silylester-Methode [vgl. Übersicht: E. Wünsch (1974), Synthese von Peptiden, Houben-Weyl, Methoden der organischen Chemie, Bände 15/1 und 15/2].

Eine Variante der Aktivester-Methode nach Birkofer et al. führt praktisch racemisierungsfrei zum gewünschten Endprodukt (Birkofer L. und Ritter, A.: Die Silylierung als Hilfsmittel in der organischen Synthese, in: Neuere Methoden der präparativen organischen Chemie, Bd. II, S. 185-209). Die Dipeptidsynthese mit einem N-geschützten D-2-Phenylglycin und D-2-Phenylglycinmethylester nach der Methode der gemischten Anhydride führt dagegen bei der Stufe der notwendigen Esterverseifung zur völligen Racemisierung.

Die vorliegende Erfindung betrifft weiterhin pharmazeutische Präparate, welche die Verbindung der Formel I oder ein Salz bzw. Säureadditionssalz enthalten. Bei den erfindungsgemässen pharmazeutischen Präparaten handelt es sich um solche zur enteralen, wie oralen oder rektalen, sowie parenteralen Verabreichung, welche die pharmakologischen Wirkstoffe allein oder zusammen mit einem üblichen pharmazeutisch anwendbaren Trägermaterial enthalten. Die erfindungsgemässen Verbindungen, insbesondere das Dipeptid-Hydrochlorid kann in Form von injizierbaren Lösungen eingesetzt werden, insbesondere für die lokale Applikation, d.h. zur intratumoralen Applikation.

Vorteilhafterweise liegt die pharmazeutische Zubereitung des Wirkstoffes in Form von Einzeldosen vor, die auf die gewünschte Verabreichung abgestimmt sind, wie z.B. Tabletten, Dragées, Kapseln, Suppositorien, Granulate, Emulsionen oder Suspensionen. Solche Lösungen sind vorzugsweise wässrige Lösungen, wobei diese z.B. lyophilisierten Präparate, welche die Wirksubstanz allein oder zusammen mit einem Trägermaterial enthalten, vor Gebrauch hergestellt werden können. Die Dosierung liegt zwischen 1-1000 mg/Dosis, vorzugsweise bei 200-400 mg/Dosis.

Nach einer lokalen Applikation, z.B. in gesunde Meerschweinchenhaut wurde eine massive Infiltration mit Makrophagen und Riesenzellen am Ort der Applikation beobachtet. Eine lokale und scharf abgegrenzte Gewebseinschmelzung ist die Folge. Auch eine Gewebseinschmelzung bei intakter Hautoberfläche wird gesehen. Bemerkenswert ist hierbei, dass keine Anzeichen einer allgemeinen Unverträglichkeit beobachtet werden. Die DL 50 der erfindungsgemässen Verbindung beträgt im akuten Versuch 1000 mg/kg i.v. (Nager). Zum Nachweis der spezifisch gewebsauflösenden Wirkung der erfindungsgemässen Verbindung wurde Meerschweinchen einmalig D-2-Phenylglycyl-D-2-phenylglycin unter die rasierte Rückenhaut appliziert. Die Dosierungen betrugen 25 bzw. 50 mg/Tier. Vergleichsweise wurde zur 25-mg-Dosis Glycin-hydrobromid

als Kontrolle (10,7 mg/Tier) sowie das Racemat DL-2-Phenylglycyl-DL-2-phenylglycin (25 mg/Tier) eingesetzt.

Die Tiere wurden über 7 Tage auf klinische sowie lokale Veränderungen beobachtet. Es folgte eine Sektion mit pathologisch-anatomischer und pathalogisch-histologischer Beurteilung der Injektionsstelle (Haut und Muskulatur).

Die Injektion der erfindungsgemässen Verbindungen bewirkt eine Gewebseinschmelzung durch hochgradige Makrophagen- und Riesenzellinfiltration. Es handelt sich hierbei um eine chemotaktische und aktivierende Wirkung auf Makrophagen, die auch tumorauflösend wirkt. Systemisch toxische Erscheinungen oder klinisch fassbare Veränderungen konnten bei keinem Versuchstier festgestellt werden. Die Applikation der Kontrollverbindung sowie des Racemats bewirkt hingegen keine lokalen Veränderungen.

Die Erfindung wird anhand nachstehender Beispiele näher beschrieben:

*Beispiel 1*

*Darstellung von D-2-Phenylglycyl-D-2-phenylglycin-hydrobromid*

N-Benzyloxycarbonyl-D-2-phenylglycin, Fp. 134°C, $[\alpha]_D^{20}$ = —110,9° (c = 1/MeOH) wird in Tetrahydrofuran bei +4°C mit 4-Nitrophenyol in Gegenwart von N,N'-Dicyclohexylcarbodiimid zum Nitrophenylester umgesetzt. Fp. 102°C, $[\alpha]_D^{20}$ = —66,2° (c = 1/MeOH). Den Nitrophenylester verschmilzt man zwischen 60°C und 100°C mit N-Trimethylsilylacetamid und D-2-Phenylglycin zum N-Benzyloxycarbonyl-dipeptidsilylester und erhält nach Aufarbeitung N-Benzyloxycarbonyl-D-2-phenylglycyl-D-2-phenylglycin, Fp. 210°C, $[\alpha]_D^{20}$ = —90,3° (c = 1/DMF). Die Demaskierung erfolgt mit HBr/Eisessig und führt zum D-2-Phenylglycyl-D-2-phenylglycin-hydrobromid, Fp. ab 175°C (Z), $[\alpha]_D^{20}$ = —111,7° (c = 1/$H_2O$) $C_{16}H_{16}N_2O_3 \cdot HBr$ [365,2].

*Beispiel 2*

*Darstellung von D-2-Phenylglycyl-D-2-phenylglycin-hydrochlorid*

*Stufe 1*

N-tert.-Butoxycarbonyl-D-2-phenylglycin

75,6 g D-2-Phenylglycin ≙0,5 Mol, Fp. 302°C (Z), $[\alpha]_D^{20}$ = —154,4° (c = 1/1nHCl) werden in einer Lösung von 20 g NaOH (≙0,5 Mol) in 500 ml Wasser gelöst, im Eis-Wasserbad auf +4°C abgekühlt und unter Rühren mit einer Lösung von 120,1 g Di-tert.-butyldicarbonat (≙0,55 Mol) in 1000 ml Dioxan versetzt. Nach Beendigung der Di-tert.-butyldicarbonat-Zugabe lässt man bei Raumtemperatur noch 10 Stunden nachrühren, bis die über einen Blasenzähler kontrollierte $CO_2$-Entwicklung beendet ist. Nach Ansäuern des Kolbeninhalts mit verdünnter Salzsäure bis pH 2 extrahiert man das N-tert.-Butoxycarbonyl-D-2-phenylglycin mit Essigester und schüttelt die Essigesterphase 3mal mit 100 ml Wasser aus. Die organische Phase wird sodann über $Na_2SO_4$ getrocknet, filtriert und das Filtrat im Vakuum eingeengt. Es verbleibt ein Öl, von dem eine kleine Probe mit Petrolether (40-60°C) zur Kristallisation gebracht wird. Die Hauptmenge wird nun ebenfalls mit Petrolether und den Impfkristallen versetzt und kräftig gerührt. Die Kristallisation ist nach kurzer Zeit beendet. Nach dem Absaugen und Trocknen im Vakuum erhält man 112,4 g (= 89,5% der Theorie) N-tert.-Butoxycarbonyl-D-2-phenylglycin, Fp. 90°-91°C, $[\alpha]_D^{20}$ = —129,2° (c = 1/DMF), $C_{13}H_{17}NO_4$ [251,3].

*Stufe 2*

N-tert.-Butoxycarbonyl-D-2-phenylglycin-4-nitrophenylester

125,7 g N-tert.-Butoxycarbonyl-D-2-phenylglycin (≙0,5 Mol) werden zusammen mit 69,6 g 4-Nitrophenol (≙0,5 Mol) in 800 ml Tetrahydrofuran gelöst. Nach Abkühlen des Kolbeninhalts in einem Eiswasserbad auf +4°C fügt man unter Rühren eine Lösung von 103,2 g Dicyclohexylcarbodiimid (≙0,5 Mol) in 100 ml Tetrahydrofuran hinzu, lässt auf Raumtemperatur kommen und ca. 12 Stunden nachrühren. Vom ausgefallenen Dicyclohexylharnstoff wird abgesaugt, das Filtrat im Vakuum eingeengt und der erstarrte Rückstand mit Di-iso-propylester ausgerührt. Nach dem Absaugen und Trocknen der Substanz im Vakuum erhält man 140,6 g (= 75,5% d. Theorie) N-tert.-Butoxycarbonyl-D-2-phenylglycin-4-nitrophenylester. $C_{19}H_{20}N_2O_6$ [372,4], Fp. 159° bis 160°C, $[\alpha]_D^{20}$ = —81,5° (c = 1/THF).

*Stufe 3*

N-tert.Butoxycarbonyl-D-2-phenylglycyl-D-2-phenylglycin

223,4 g N-tert.-Butoxycarbonyl-D-2-phenylglycin-4-nitrophenylester (= 0,6 Mol), 236 g N-Trimethylsilylacetamid (= 1,8 Mol) und 90,7 g D-2-Phenylglycin (= 0,6 Mol) werden unter Ausschluss von Luftfeuchtigkeit bei einer Badtemperatur von 60° bis 80°C geschmolzen und dann noch weitere 14 Stunden bei dieser Temperatur gerührt. Nach dem Erkalten nimmt man die erstarrte Schmelze in Essigester auf und schüttelt den Essigester-Extrakt mit einer gesättigten wässrigen $KHCO_3$-Lösung aus. Dabei wird desilyliert und das Kaliumsalz der N-tert.-Butoxycarbonyl-dipeptidsäure löst sich in der Wasserphase. Die abgetrennte Wasserphase schüttelt man nach Ansäuern mit verdünnter Salzsäure bis pH 2 mit frischem Essigester aus, wäscht die Essigesterphase 2mal mit kleinen Mengen Wasser und trocknet sie kurz über $Na_2SO_4$. Vom $Na_2SO_4$ wird abfiltiert und das Filtrat mit der gleichen Menge Petrolether (40° bis 60°C) versetzt. Die auskristallisierte N-tert.-Butoxycarbonyl-dipeptidsäure wird abgesaugt und im Vakuum getrocknet. Ausbeute: 170,6 g (≙74% d. Theorie) N-tert.-Butoxycarbonyl-D-2-phenylglycyl-D-2-phenylglycin. $C_{21}H_{24}N_2O_5$ [384,4], Fp. 101°C, $[\alpha]_D^{20}$ = —111,3° (c = 1/DMF).

*Stufe 4*

D-2-Phenylglycyl-D-2-phenylglycin-hydrochlorid

71,0 g N-tert.-Butoxycarbonyl-D-2-phenylglycyl-D-2-phenylglycin (≙0,185 Mol) werden in 150 ml Eisessig suspendiert und mit 300 ml einer gesättig-

ten Lösung von HCl in Eisessig unter Rühren bei Raumtemperatur versetzt. Die N-tert.-Butoxycarbonyl-dipeptidsäure geht dabei sogleich in Lösung. Kurze Zeit später beginnt bereits die Kristallisation des demaskierten Dipeptids in Form des Dipepdit-Hydrochlorids. Nach ca. 3 Stunden wird das nicht hygroskopische Salz abgesaugt, mit absolutem Ether gewaschen und 2mal aus Methanol-Ether umkristallisiert. Die bei 50°C Badtemperatur im Ölpumpenvakuum bis zur Gewichtskonstanz getrocknete Substanz wird sodann in aqua bidest. gelöst (4%ig), steril filtriert und lyophilisiert. Ausbeute: 48,2 g (≙81,2% d. Theorie) D-2-Phenylglycyl-D-2-phenylglycin-hydrochlorid. $C_{16}H_{16}N_2O_3 \cdot HCl$ [320,8], Fp. 175°C (Z), $[\alpha]_D^{20}$ = —124,1° (c = 1/Wasser)
MS-Spektrum, Varian MAT-311-A (70eV)
$M^+$—18 ($C_{16}H_{14}N_2O_2^+$): m/z 266; $M^+$—46 ($C_{15}H_{14}N_2O^+$): m/z 238; $M^+$—61: m/z 223; $C_8H_6O^+$: m/z 118; $C_7H_8N^+$: m/z 106; $C_6H_5^+$: m/z 77; $HCl^+$: m/z 36 und 38.

*Beispiel 3*

Eine 4%ige Lösung von D-2-Phenylglycyl-D-2-phenylglycin-hydrochlorid in bidestilliertem Wasser wird steril filtriert, lyophilisiert und das Lyophilisat unter sterilen Bedingungen in 100 mg, 200 mg und 400 mg-Portionen in Ampulen abgefüllt.

*Beispiel 4*

50 mg D-2-Phenylglycyl-D-2-phenylglycin-hydrochlorid, 150 mg mikrokristalline Cellulose, 50 mg Aerosil, 15 mg Cutina HR, 20 mg Hydroxymethylcellulose-phthalat. Die aufgeführten Stoffe werden gemischt, gepresst und die Presslinge mit einem Film Hydroxymethylcellulose-phthalat überzogen.

*Beispiel 5*

100 mg D-2-Phenylglycyl-D-2-phenylglycin-hydrochlorid, 5 mg Talkum, 10 mg Aerosil 200 werden gemischt, granuliert und in Hartgelatinekapseln abgefüllt.

**Patentansprüche für die Vertragsstaaten:**
BE, CH, FR, GB, IT, LI, LU, NL, SE

1. Optisch aktives Dipeptid D-2-Phenylglycyl-D-2-phenylglycin der Formel I

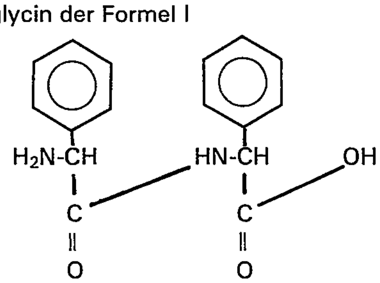

I

und seine pharmazeutisch verträglichen Salze und Säureadditionssalze.

2. D-2-Phenylglycyl-D-2-phenylglycin-hydrobromid und -hydrochlorid.

3. Verfahren zur Herstellung von Verbindungen der Formel I gemäss Anspruch 1 oder 2, dadurch gekennzeichnet, dass man in an sich bekannter Weise den durch tert.-Butoxycarbonyl N-geschützten p-Nitrophenylester des D-2-Phenylglycins in Gegenwart von N-Trimethylsilylacetamid mit D-2-Phenylglycin umsetzt und aus dem erhaltenen Produkt in Gegenwart der entsprechenden Säure die tert.-Butoxycarbonylgruppe abspaltet.

4. Arzneimittelzubereitungen, dadurch gekennzeichnet, dass sie die Verbindung gemäss Formel I oder ein pharmazeutisch verträgliches Salz bzw. Säureadditionssalz gemäss Anspruch 1 oder 2 zusammen mit einem pharmazeutisch geeigneten Verdünnungsmittel oder Trägermaterial enthalten.

5. Arzneimittelzubereitungen, dadurch gekennzeichnet, dass sie ein pharmazeutisch verträgliches Salz bzw. Säureadditionssalz gemäss Anspruch 1 oder 2 zusammen mit einem pharmazeutisch geeigneten Verdünnungsmittel oder Trägermaterial in einer für die lokale Applikation geeignete Form enthalten.

**Patentanspruch für den Vertragsstaat:** AT

Verfahren zur Herstellung des optisch aktiven Dipeptids D-2-Phenylglycyl-D-2-phenylglycin der Formel I

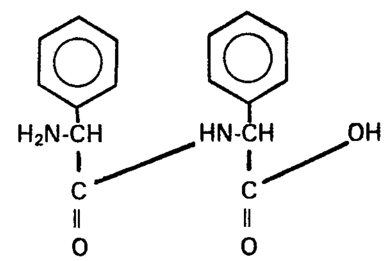

I

und seiner pharmazeutisch verträglichen Salze und Säureadditionssalze, dadurch gekennzeichnet, dass man in an sich bekannter Weise den durch tert.-Butoxycarbonyl N-geschützten p-Nitrophenylester des D-2-Phenylglycins in Gegenwart von N-Trimethylsilylacetamid mit D-2-Phenylglycin umsetzt und aus dem erhaltenen Produkt in Gegenwart der entsprechenden Säure, vorzugsweise der Brom- oder Chlorwasserstoffsäure, die tert.-Butoxycarbonylgruppe abspaltet.

**Claims for the Contracting States:**
BE, CH, FR, GB, IT, LI, LU, NL, SE

1. Optically active dipeptide D-2-phenyl glycyl-D-2-phenyl glycin according to formula I

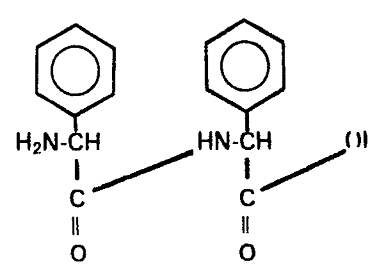

I

and its pharmaceutically compatible salts and acid addition salts.

2. D-2-phenyl glycyl-D-2-phenyl glycin-hydrobromide and hydrochloride.

3. Process for the preparation of compounds according to formula I as defined in either of claims 1 or 2, characterized in that there is reacted in a manner known per se the tert.-butoxy-carbonyl N protected p-nitrophenyl ester of D-2-phenyl glycin in the presence of N-trimethyl silyl acetamide with D-2-phenyl glycin and cracked from the obtained product in the presence of the appropriate acid the tert.-butoxy carbonyl group.

4. Pharmaceutical compositions, characterized in that they contain the compound according to formula I or a pharmaceutically compatible salt or acid addition salt as defined in either of claims 1 and 2 together with a pharmaceutically appropriate thinner or carrier.

5. Pharmaceutical compositions, characterized in that they contain a pharmaceutically compatible salt or acid addition salt as defined in either of claims 1 or 2 together with a pharmaceutically appropriate thinner or carrier material in a form appropriate for local application.

**Claim for the Contracting State:** AT

Process for the production of compounds of formula I

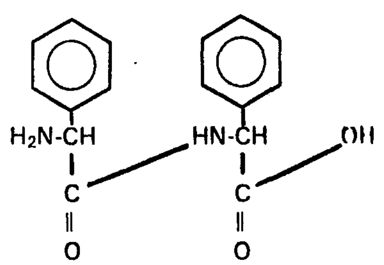

I

and its pharmaceutically compatible salts and acid addition salts, characterized in that the p-nitrophenylester of D-2-phenylglycin protected at its nitrogen atom by a tert.-butoxycarbonyl is subjected to reaction with D-2-phenylglycin in a manner known per se in the presence of N-trimethylsilylacetamide and from the resulting products there is split-off the tert.-butoxycarbonyl group in the presence of the corresponding acid, preferably the hydrobromic or hydrochlorid acid.

**Revendications pour les Etats Contractants:** BE, CH, FR, GB, IT, LI, LU, NL, SE

1. Le dipeptide optiquement actif D-2-phénylglycyl-D-2-phénylglycine de formule I

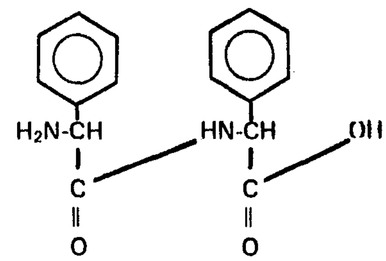

I

et ses sels et sels d'addition d'acides pharmaceutiquement acceptables.

2. Bromhydrate et chlorhydrate de D-2-phénylglycyl-D-2-phénylglycine.

3. Procédé de préparation de composés de formule I, selon l'une des revendications 1 et 2, caractérisé en ce qu'on fait réagir de façon connue le p-nitrophénylester de la D-2-phénylglycine N-protégé par un tert.-butoxycarbonyle en présence de N-triméthylsilylacétamide avec de la D-2-phénylglycine et en ce qu'on sépare le groupe tert.-butoxycarbonyle du produit obtenu en présence de l'acide correspondant.

4. Préparations médicamenteuses, caractérisées en ce qu'elles contiennent le composé selon la formule I ou un sel ou selon les cas sel d'addition d'acides pharmaceutiquement acceptable selon l'une des revendications 1 et 2 avec un diluant ou support pharmaceutiquement approprié.

5. Préparations médicamenteuses, caractérisées en ce qu'elles contiennent un sel ou selon les cas sel d'addition d'acides pharmaceutiquement acceptable selon l'une des revendications 1 et 2 avec un diluant ou support pharmaceutiquement approprié sous une forme appropriée à l'application locale.

**Revendication pour l'Etat Contractant:** AT

Procédé pour la préparation des composés de formule I

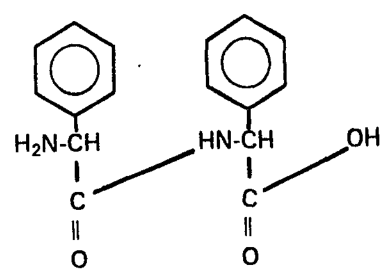

I

et ses sels et sels d'addition d'acides pharmaceutiquement acceptables, caractérisé en ce que, de manière en soi connue, on fait réagir le p-nitrophénylester de la D-2-phénylglycine N-protégé par un tert.-butoxycarbonyle en présence de N-triméthyl-silylacétamide avec de la D-2-phénylglycine et, en ce qu'on sépare le groupe tert.-butoxycarbonyle du produit obtenu en présence de l'acide correspondant, de préférence le bromhydrate ou le chlorhydrate.